Europäisches Patentamt

European Patent Office

Office européen des brevets

(11) Veröffentlichungsnummer: **0 017 006**
**B1**

(12) **EUROPÄISCHE PATENTSCHRIFT**

(19)

(45) Veröffentlichungstag der Patentschrift: **22.02.84**

(21) Anmeldenummer: **80101179.2**

(22) Anmeldetag: **07.03.80**

(51) Int. Cl.³: **A 61 F 13/02, A 61 B 10/00**

(54) Epikutantestpflaster.

(30) Priorität: **08.03.79 DE 2909071**

(43) Veröffentlichungstag der Anmeldung:
**15.10.80 Patentblatt 80/21**

(45) Bekanntmachung des Hinweises auf die Patenterteilung:
**22.02.84 Patentblatt 84/8**

(84) Benannte Vertragsstaaten:
**AT BE CH DE FR GB IT LU NL SE**

(56) Entgegenhaltungen:
**CH - A - 373 140**
**DE - A - 2 444 379**
**DE - A - 2 755 838**
**DE - C - 146 315**
**US - A - 2 190 745**
**US - A - 2 235 436**
**US - A - 3 212 495**
**US - A - 3 894 531**

(73) Patentinhaber: **Schmücking, Carl-Günther, Dr. med.**
**Caspar-Voght-Strasse 79**
**D-2000 Hamburg 26 (DE)**

(72) Erfinder: **Schmücking, Carl-Günther, Dr. med.**
**Caspar-Voght-Strasse 79**
**D-2000 Hamburg 26 (DE)**

(74) Vertreter: **Vossius Vossius Tauchner Heunemann Rauh**
**Siebertstrasse 4 P.O. Box 86 07 67**
**D-8000 München 86 (DE)**

Courier Press, Leamington Spa, England.

Epikutantestpflaster

Die Erfindung betrifft Epikutantestpflaster mit mindestens einem Testzentrum, das auf einer überstehenden, einen Rand bildenden Pflasterfolie befestigt ist.

Epikutanteste dienen insbesondere zur Aufklärung der Ursachen von chronischen allergischen Kontaktekzemen. Zu diesem Zweck wird die zu prüfende Testsubstanz auf ein geeignet präpariertes Testpflaster aufgetragen, das dann vom Arzt oder dessen Helfer auf die Haut des Patienten, beispielsweise auf dessen Arm oder Rücken, appliziert wird. Die Applikationsdauer beträgt gewöhnlich 24 Stunden. Die erste Ablesung der Reaktion durch den Hautarzt erfolgt unmittelbar nach Abnahme der Testpflaster; weitere Ablesungen der Reaktion erfolgen 24 und/oder 48 Stunden später, wobei für den Hautarzt ein Problem darin besteht, die jeweiligen Teststellen wiederzufinden. Zu diesem Zweck muß die Lage der Testzentren auf der Haut des Patienten zusätzlich markiert werden, und dies geschieht üblicherweise in der Form, daß der Arzt oder dessen Helfer nach Abzug des Testpflasters die Lage der Testzentren direkt auf dem Körper des Patienten mit Hilfe eines Stiftes markiert. Da sich diese Markierung jedoch im Laufe der Zeit abreibt bzw. verwischt, muß sie wiederholt erneuert werden. Abgesehen von einer Verschmutzung der Wäsche können diese Markierungen Hautreizungen hervorrufen, die gegebenenfalls die Testreaktionen verfälschen können.

Eine Markierung kann auch in der Weise vorgenommen werden, daß ein entsprechend markierter Pflasterstreifen neben das Testpflaster geklebt und während der gesamten Ablesezeit auf der Haut des Patienten belassen wird. Dabei wird zwar der Patient nicht in Mitleidenschaft gezogen, doch ist dieses Vorgehen kosten- und zeitintensiv.

Aus der DE—A1—2 755 838 ist ein Epikutantestpflaster bekannt, bei dem ein Scheibchen aus absorptionsfähigem Material zur Aufnahme der Testsubstanz auf ein größeres Stück Haftgewebe aufgeschweißt ist, so daß um das Scheibchen herum ein Rand des Haftgewebes gebildet wird. Dieser mit einem Haftstoff belegte Rand des Haftgewebes ist zum Fixieren des bekannten Epikutantestpflasters auf der Haut des Patienten vorgesehen. Vor der Benutzung ist das Testpflaster mit einer Schutzfolie versehen, die durch den Haftstoff auf dem Rand festgehalten und vor dem Gebrauch des Pflasters abgezogen werden kann.

Aus der DE—A1—2 444 379, insb. Fig. 4 mit zugehöriger Beschreibung, ist ein Epikutantestpflaster bekannt, bei dem ein Markierungsfeld innerhalb von Markierungslinien liegt. Diese Markierung kann sich jedoch im Laufe der Zeit abreiben oder verwischen, so daß eine genaue Lokalisierung der Testbereiche nach einer gewissen Zeit nicht mehr möglich ist. Entsprechendes gilt auch für die Beschriftung mittels einer Farbsubstanz in dafür vorgesehenen Beschriftungsfeldern neben den Testbereichen.

Der Erfindung liegt die Aufgabe zugrunde, ein Epikutantestpflaster der eingangs beschriebenen Art zu schaffen, das bei einfacher Handhabung eine exakte Markierung der Teststellen auf der Haut des Patienten ermöglicht.

Diese Aufgabe wird erfindungsgemäß dadurch gelöst, daß der Rand der Pflasterfolie durch eine Solltrennlinie an dem das Testzentrum enthaltenden Teil der Pflasterfolie lösbar befestigt ist, d.h., der Rand wird mit einer Solltrennlinie, z.B. in Form von Perforationen und/oder mit einem Reißfaden, versehen, so daß beim Abnehmen des Teils des Pflasters mit der Testsubstanz zumindestes ein Teil des Pflasterrandes auf der Haut des Patienten verbleibt und hieraus auch zu einem späteren Zeitpunkt noch die genaue Lage des Testzentrums auf der Haut ermittelt werden kann.

Weitere erfindungsgemäße Ausführungsformen ergeben sich aus den Unteransprüchen sowie der nachstehenden Figurenbeschreibung unter Bezug auf die anliegende Zeichnung. Es zeigen:

Fig. 1 eine perspektivische Ansicht eines erfindungsgemäßen Epikutantestpflasters mit einem Testzentrum,

Fig. 2a und 2b Aufsichten von Epikutantestepflastern mit mehreren Testzentren in jeweils einer Reihe mit umlaufender bzw. einseitiger Perforation des Randes und

Fig. 3 eine Aufsicht eines Epikutantestpflasters in Endlosform mit doppelreihig angeordneten Testzentren.

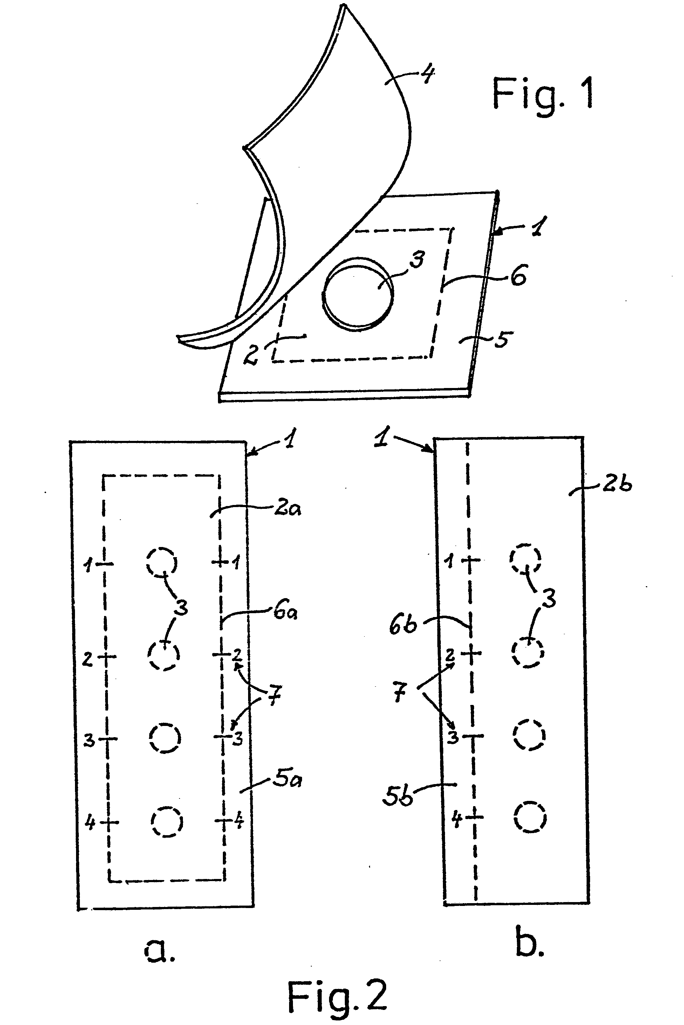

Das in Figur 1 dargestellte Epikutantestpflaster besteht im wesentlichen aus einer Pflasterfolie 1, auf deren einen beim Gebrauch auf der Haut des Patienten aufliegenden, haftstoffbeschichteten Seite ein Testzentrum 3 angebracht ist. Dieses Testzentrum 3 befindet sich etwa in der Mitte der Pflasterfolienfläche, so daß die Pflasterfolie über das Testzentrum 3 hinaus übersteht. Dieser überstehende, haftstoffbeschichtete Teil der Pflasterfolie 1 dient zum Fixieren des Pflasters auf der Haut des Patienten. Ferner kann die haftstoffbeschichtete Seite der Pflasterfolie mit einer Schutzfolie 4, etwa aus gepreßtem Kunststoffband, versehen sein, die das Testpflaster zunächst schützt und vor dem Gebrauch abgezogen wird. Das Testzentrum 3 besteht etwa aus einem kreisförmigem Scheibchen aus absorptionsfähigem Material oder aus einem Metallschälchen zur Aufnahme der Testsubstanz.

In dem gegenüber dem Testzentrum 3 überstehenden Teil der Pflasterfolie 1 sind Solltrenn- oder Sollreißlinien 6 vorgesehen, mit deren Hilfe der innere Teil 2 der Pflasterfolie mit dem Testzentrum 3 nach ausreichender Applikation auf der Haut des Patienten herausgelöst werden kann, so daß der Rand 5 in Form eines Rahmens auf der Haut verbleibt und auch Tage nach der Abnahme des Testzentrums 3 eine genaue Lokalisierung der Teststelle zur Ermittlung eventueller Spätreaktionen möglich ist.

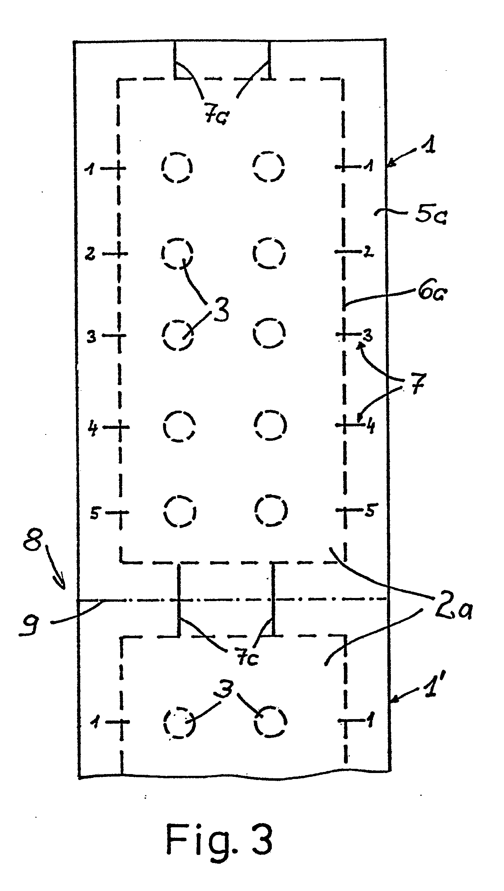

Die weiteren Figuren 2 und 3 zeigen Aufsichten weiterer Ausführungsformen von der der Haut des Patienten abgewandten Seite. Bei den in den Figuren 2a und 2b dargestellten Ausführungsformen sind mehrere Testzentren 3 in einer einzigen Reihe im Abstand zueinander angeordnet, um gleichzeitig mehrere Epikutantests mit verschiedenen Testsubstanzen durchzuführen. Bei der Ausführungsform der Figur 2a werden alle Testzentren 3 von einer gemeinsamen, etwa rechteckig verlaufenden Solltrennlinie 6a umgeben; auf dem ebenfalls etwa rechteckigen, gemeinsamen Rand 5a sind Markierungen 7 vorgesehen, um nach dem Heraustrennen des Innenteils 2a mit den Testzentren 3 die einzelnen Teststellen lokalisieren zu können.

Bei der Ausführungsform der Figur 2b ist lediglich auf der einen Seite in Längsrichtung des Teststreifens 2b ein Rand 5b mit entsprechenden Markierungen 7 vorgesehen.

Die in Figur 3 dargestellte Ausführungsform des erfindungsgemäßen Epikutantestpflasters ist ähnlich der in Figur 2a, jedoch sind die Testzentren in zwei zueinander parallelen Reihen angeordnet. Auf dem durch die Solltrennlinie 6c abgeteilten Rand 5c sind auf der Seite ebenfalls wie bei Figur 2a und Figur 2b Markierungen 7 vorgesehen, während die Lokalisierung der beiden Reihen der Teststellen nach dem Entfernen des Innenteils 2a durch Markierungen 7c am oberen und unteren Randabschnitt des Randes 5c ermöglicht wird.

Gemäß Figur 3 können die erfindungsgemäßen Epikutantestpflaster in Endlosform aneinander gereiht werden, d.h. ein Pflasterband 8 besteht aus mehreren gleichartigen Testpflastern 1, 1', die etwa an ihrer Schmalseite miteinander verbunden sind und durch eine Solltrennlinie 9, beispielsweise durch Perforationen, vor der Benutzung leicht einzeln abgetrennt werden können. Eine entsprechende Endlosanordnung der anderen erfindungsgemäßen Ausführungsformen ist ebenfalls möglich.

Die Markierungen 7, 7c, können auf der der Haut des Patienten abgewandten Seite des Pflasters beispielsweise aufgedruckt, eingeprägt oder eingekerbt sein.

Da beim Abnehmen des Pflasterteils mit den Testzentren von der Haut des Patienten der Rand, gegebenenfalls mit Markierungen, auf der Haut des Patienten verbleibt, können auch nach Tagen noch die Teststellen genau lokalisiert werden.

## Patentansprüche

1. Epikutanpflaster mit mindestens einem Testzentrum (3), das auf einer überstehenden, einen Rand (5) bildenden Pflasterfolie (1) befestigt ist, dadurch gekennzeichnet, daß der Rand (5) der Pflasterfolie (1) durch eine Solltrennlinie (6) an dem das Testzentrum (3) enthaltenden Teil (2) der Pflasterfolie (1) lösbar befestigt ist.

2. Epikutantestpflaster nach Anspruch 1, dadurch gekennzeichnet, daß die Solltrennlinie (6) durch Perforationen gebildet ist.

3. Epikutantestpflaster nach Anspruch 1 oder 2, dadurch gekennzeichnet, daß die Solltrennlinie (6) einen Reißfaden aufweist.

4. Epikutantestpflaster nach einem der Ansprüche 1 bis 3, dadurch gekennzeichnet, daß der Rand (5) Markierungen (7) aufweist.

5. Epikutantestpflaster nach Anspruch 4, dadurch gekennzeichnet, daß die Markierungen aufgedruckt, eingekerbt oder eingeprägt sind.

6. Epikutantestpflaster nach einem der Ansprüche 1 bis 5, dadurch gekennzeichnet, daß der Rand (5) und die Solltrennlinie (6) das Testzentrum (3) umgibt.

7. Epikutantestpflaster nach einem der Ansprüche 1 bis 6, dadurch gekennzeichnet, daß die Testzentren (3) reihenförmig angeordnet sind.

8. Epikutantestpflaster nach Anspruch 7, dadurch gekennzeichnet, daß die Testzentren (3) in Mehrfachreihen, vorzugsweise in Doppelreihen, angeordnet sind.

9. Epikutantestpflaster nach einem der Ansprüche 1 bis 8 als Endlospflaster, dadurch gekennzeichnet, daß zwischen den aneinander gereihten Teilpflastern Sollreißlinien (9) vorgesehen sind.

## Revendications

1. Emplâtre pour teste épicutanés comportant au moins un centre de test (3) qui est fixé sur une feuille d'emplâtre (1) saillante et formant un bord (5), caractérisé par le fait que le bord (5) de la feuille d'emplâtre (1) est fixé de façon détachable par une ligne de séparation imposée (6) sur la partie (2) de la feuille d'emplâtre (1) comportant le centre de test (3).

2. Emplâtre pour tests épicutanés selon la revendication 1, caractérisé par le fait que la ligne de séparation imposée (6) est formée par des perforations.

3. Emplâtre pour tests épicutanés selon la revendication 1 ou 2, caractérisé par le fait que la ligne de séparation imposée (6) comporte un fil d'arrachement.

4. Emplâtre pour tests épicutanés selon l'une des revendications 1 à 3, caractérisé par le fait que le bord (5) comporte des marquages (7).

5. Emplâtre pour tests épicutanés selon la revendication 4, caractérisé par le fait que les marquages sont imprimés, entaillés ou empreints.

6. Emplâtre pour tests épicutanés selon l'une des revendications 1 à 5, caractérisé par le fait que le bord (5) et la ligne de séparation imposée (6) entourent le centre de tests (3).

7. Emplâtre pour tests épicutanés selon l'une des revendications 1 à 6, caractérisé par le fait que les centres de tests (3) sont disposés en rangées.

8. Emplâtre pour tests épicutanés selon la revendication 7, caractérisé par le fait que les centres de tests (3) sont disposés en rangées multiples, de préférence en rangées doubles.

9. Emplâtre pour tests épicutanés selon l'une des revendications 1 à 8 en tant qu'emplâtre sans fin, caractérisé par le fait qu'entre les emplâtres partiels successifs on prévoit des lignes de séparation imposées (9).

## Claims

1. A plaster for epicutaneous tests which has at least one test center (3) and is fixed onto a projecting plaster film (1) forming a rim (5), characterized in that thanks to the desired separation line (6), the rim (5) of the plaster film (1) is detachably fixed to that part (2) of the plaster film (1) which contains the test center (3).

2. The epicutaneous test plaster according to claim 1, characterized in that the desired separation line (6) is formed by perforations.

3. The epicutaneous test plaster according to claim 1 or 2, characterized in that the desired separation line (6) exhibits a rip cord.

4. The epicutaneous test plaster according to one of claims 1 to 3, characterized in that the rim (5) exhibits markings (7).

5. The epicutaneous test plaster according to claim 4, characterized in that the markings are printed, notched or impressed.

6. The epicutaneous test plaster according to one of claims 1 to 5, characterized in that the rim (5) and the desired separation line (6) surrounds the test center (3).

7. The epicutaneous test plaster according to one of claims 1 to 6, characterized in that the test centers (3) are arranged in rows.

8. The epicutaneous test plaster according to claim 7, characterized in that the test centers (3) are arranged in multiple rows, preferably in double rows.

9. The epicutaneous test plaster according to one of claims 1 to 8 in the form of an endless plaster, characterized in that the desired separation lines (9) are provided between the partial plasters stringed together.

0 017 006

Fig. 1

Fig. 2

a.

b.

Fig. 3